# EUROPEAN PATENT SPECIFICATION

(11) **EP 3 684 079 B1**
(45) Date of publication and mention of the grant of the patent: **20.03.2024**
(21) Application number: 19166417.6
(22) Date of filing: 29.03.2019
(51) Int. Cl.: H04R 25/00, A61B 5/11, A61B 5/00

(54) **HEARING DEVICE FOR ORIENTATION ESTIMATION AND METHOD OF ITS OPERATION**
HÖRGERÄT ZUR AUSRICHTUNGSSCHÄTZUNG UND VERFAHREN ZUM BETRIEB DAVON
DISPOSITIF AUDITIF POUR ESTIMATION D'ORIENTATION ET SON PROCÉDÉ DE FONCTIONNEMENT

(43) Date of publication of application: 22.07.2020
(73) Proprietor: Sonova AG, 8712 Stäfa (CH)
(72) Inventor: EL GUINDI, Nadim, 8049 Zürich (CH); STUMPF, Nina, 8708 Männedorf (CH); SIGWANZ, Ullrich, 8634 Hombrechtigkon (CH)

(56) References cited:
- US-A1- 2013 343 584
- US-A1- 2015 230 036
- US-A1- 2016 278 647
- US-A1- 2017 258 374
- US-A1- 2018 125 423
- BLANCA FLORENTINO-LIANO ET AL: "Long term human activity recognition with automatic orientation estimation", MACHINE LEARNING FOR SIGNAL PROCESSING (MLSP), 2012 IEEE INTERNATIONAL WORKSHOP ON, IEEE, 23 September 2012 (2012-09-23), pages 1-6, XP032470885, DOI: 10.1109/MLSP.2012.6349789 ISBN: 978-1-4673-1024-6
- VAN LAARHOVEN SIMON N ET AL: "Validation of a novel activity monitor in impaired, slow-walking, crutch-supported patients", ANNALS OF PHYSICAL AND REHABILITATION MEDICINE, ELSEVIER, AMSTERDAM, NL, vol. 59, no. 5, 19 September 2016 (2016-09-19), pages 308-313, XP029829705, ISSN: 1877-0657, DOI: 10.1016/J.REHAB.2016.05.006
- SURAPA THIEMJARUS: "A Device-Orientation Independent Method for Activity Recognition", BODY SENSOR NETWORKS (BSN), 2010 INTERNATIONAL CONFERENCE ON, IEEE, PISCATAWAY, NJ, USA, 7 June 2010 (2010-06-07), pages 19-23, XP031705031, ISBN: 978-1-4244-5817-2

## Description

### TECHNICAL FIELD

This disclosure relates to a hearing device comprising a housing configured to be worn at a wearing position at an ear of a user, and an inertial sensor included in the housing, according to the preamble of claim 1. The disclosure further relates to a method of operating a hearing device comprising such a hearing device, according to the preamble of claim 12. 5.

### BACKGROUND

Hearing devices may be used to improve the hearing capability or communication capability of a user, for instance by compensating a hearing loss of a hearing-impaired user, in which case the hearing device is commonly referred to as a hearing instrument such as a hearing aid, or hearing prosthesis. A hearing device may also be used to produce a sound in a user's ear canal. Sound may be communicated by a wire or wirelessly to a hearing device, which may reproduce the sound in the user's ear canal. Hearing devices are often employed in conjunction with communication devices, such as smartphones, for instance when listening to sound data processed by the communication device and/or during a phone conversation operated by the communication device. More recently, communication devices have been integrated with hearing devices such that the hearing devices at least partially comprise the functionality of those communication devices.

Different types of hearing devices can be distinguished by the position at which they are intended to be worn at an ear of a user. Some types of hearing devices can comprise a behind-the-ear part (BTE part) comprising a housing configured to be worn at a wearing position behind the ear of the user. The housing of the BTE part can accommodate functional components of the hearing device. Hearing devices comprising a BTE part can include, for instance, receiver-in-the-canal (RIC) hearing aids and behind-the-ear (BTE) hearing aids. Other functional components of such a hearing device may be intended to be worn at a different position at the ear, in particular at an ear location at least partially inside an ear canal. For instance, a RIC hearing aid may comprise a receiver intended to be worn at least partially inside the ear canal. The receiver may be implemented in another housing, for instance an earpiece adapted for an insertion and/or a partial insertion into the ear canal. A BTE hearing aid may comprise a sound conduit intended to be worn at least partially inside the ear canal. Other types of hearing devices, for instance, earbuds, earphones, and hearing instruments such as in-the-ear (ITE) hearing aids, invisible-in-the-canal (IIC) hearing aids, and completely-in-the-canal (CIC) hearing aids, commonly comprise such a housing intended to be worn at the ear position at least partially inside the ear canal, but may not comprise a BTE part having a housing intended to be worn behind the ear. An inertial sensor may be included in a housing of a BTE part and/or in a housing intended to be worn at least partially inside the ear canal and/or in any other housing worn at the user's ear, such as a beam or bracket of a headphone.

Often it is desirable to estimate an orientation of a wearing position, at which the housing of the hearing device is worn at the ear of the user with respect to his spatial surroundings. For instance, when the user is wearing a BTE part of a hearing device behind his ear and lying down on a bed or leaning back on a sofa, there is a risk that the BTE part is very close to a bed surface, backrest, pillow and/or the like, or even is in contact with such a surface. Such a situation can provoke a significant increase of feedback, when a microphone is implemented with the BTE part. Therefore, a reduction of an amplification or gain is desirable in this situation, at least by a certain amount, in order to alleviate or prevent the feedback risk. This requires, however, a reliable recognition of the situation in which the user is in a resting position. The spatial orientation of the user's ear at the wearing position of the housing can give an indication for differing postures of the user, in particular to distinguish situations in which the user is in an upright pose from situations in which the user is in a more reclined position. The latter situation can suggest that the user is in a resting position and therefore more likely to sit on a couch or lie on a bed such that there is an increased risk of feedback gain. As another example, an estimate of the spatial ear orientation can be useful to determine more optimized parameters of the hearing device in various other life situations including, for instance, an improved directionality of a beamformer which may be implemented with a hearing device. The preferred directionality can depend on the user's listening intention which can be related to the ear orientation. As yet another example, an estimate of the ear orientation can be useful to determine user specific health data, for instance a rather imbalanced or cramped head position over a longer period, and may be evaluated, in particular, in conjunction with data provided by other health sensors implemented with the hearing device.

In order to estimate the spatial orientation of the hearing device at the user's ear, the hearing device can include an inertial sensor communicatively coupled to an orientation estimator. In this way, the orientation estimator can be configured to provide orientation data indicative of a spatial orientation of the hearing device housing containing the inertial sensor, when the housing is worn at the ear of the user. Yet the spatial orientation of the user's ear not only depends on the spatial orientation of the housing but also on the wearing position of the housing at the ear, for instance behind the ear or inside the ear canal. Variances of the wearing position hence lead to false estimations of the spatial orientation of the user's ear. In consequence, operations relying on a correct estimation of the ear orientation and/or hearing device orientation cannot be performed in a reliable way.
US 2013/0343584 A1 discloses a hearing device configured to monitor the health of a user by a plurality of medical sensors including a position/motion sensor which may also include an accelerometer. The hearing device may include a first portion shaped to be positioned behind the ear of the user and a second portion shaped to be at least partially inserted into the ear canal, wherein the position/motion sensor is provided at the second portion. The health monitoring based on the information acquired by the position/motion sensor can include information whether the user has fallen down or collapsed, wherein a voice and/or video assist of a handheld device may be used to gather feedback from the user, or information regarding a current body position, for example standing up, leaning over or lying down, or an orientation of the user, wherein the hearing device can augment or verify this information. The hearing device can also be configured to perform an audio processing providing for an enhanced audio perspective depending on detecting walking impacts and an outdoor Global Positioning System (GPS) location.
US 2016/0278647 A1 discloses a wearable sensor device such as an earbud with heart rate sensing or monitoring functionality, wherein a misfit and/or misalignment of the heart rate sensor can be detected by an alignment sensor, which may include an accelerometer. The information acquired by the accelerometer can include acceleration vectors and a gravity component. A classifier can compare the gravity component of the acceleration data to historical data, for instance the classifier may be trained using known possible orientations and/or angles of the sensor device. Information about the misalignment of the heart rate sensor can be employed to generate a notification to the user.
BLANCA FLORENTINO-LIANO ET AL disclose, in "Long term human activity recognition with automatic orientation estimation", MACHINE LEARNING FOR SIGNAL PROCESSING (MLSP), 2012 IEEE INTERNATIONAL WORKSHOP ON, IEEE, 23 September 2012 (2012-09-23), pages 1-6, an activity recognition algorithm based on a single inertial sensor that allows recognition of day to day activities, such as sitting, lying, standing, walking, running and jumping, to achieve high classification results on long term recordings of the day to day activities. The inertial sensor can be simply attached, in a "plug-and-play" manner, to a belt with no specific requirements for its exact placement and orientation. The algorithm shall be independent of the sensor orientation since, in real life situations, control of the orientation of the sensor when fixed to the belt can be compromised due to variations in body shape, clothing and other factors which affects the accuracy of the automatic activity recognition. The proposed solution to make the algorithm invariant to sensor orientation consists of, firstly, identifying walking epochs in which the user's body is aligned to a reference frame, secondly, using the walking epochs to compute an initial estimate of the orientation, and finally, performing a fine-tuning of the orientation. Identifying the walking epochs by a walking detector allows finding segments in the sensor data in which the subject wearing the sensor is upright such that its body frame is aligned to the reference frame. VAN LAARHOVEN SIMON N ET AL disclose, in "Validation of a novel activity monitor in impaired, slow-walking, crutch-supported patients", ANNALS OF PHYSICAL AND REHABILITATION MEDICINE, ELSEVIER, AMSTERDAM, NL, vol. 59, no. 5, 19 September 2016 (2016-09-19), pages 308-313, a method of activity monitoring of impaired, slow-walking, crutch supported patients. The activity is monitored by an accelerometer taped to a lateral leg of participants by using hypo-allergenic double-sided tape, wherein the accelerometer's orientation is calibrated during a period of level walking, which is manually selected, to allow a differentiation between resting periods and standing periods, identifying sit-to-stand transfers, and to distinguish ascending and descending stairs from the level walking.

US 2018/125423 A1 relates to activity monitoring wearables, and more specifically to a system and method for activity monitoring eyewear and head apparel. The system comprises an intertial measuring system for determining head orientation and activity detection. This document refers to another US patent application published as US 2017/258374 A1 which discloses the calibration of a kinematic base based on a detection of when the user is walking.

US 2015/230036 A1 discloses a hearing aid with a directional microphone which is steered depending on an orientation of the hearing aid relative to the gravity vector so that the directional microphone is focused in the horizontal direction.

### SUMMARY

It is an object of the present disclosure to avoid at least one of the above mentioned disadvantages and to provide a hearing device and/or a method of operating the hearing device allowing an estimation of the spatial orientation of the housing and/or the wearing position at which the housing is worn at the ear, in particular in a more reliable way. It is another object to employ such an orientation estimate for an improved hearing device operation, in particular such that various daily situations and/or hearing situations involving different ear orientations can be accounted for.

At least one of these objects can be achieved by a hearing device comprising the features of patent claim 1 and/or in a method of operating a hearing device comprising the features of patent claim 12. Advantageous embodiments of the invention are defined by the dependent claims.

The present disclosure proposes a hearing device comprising a housing configured to be worn at a wearing position at an ear of a user. The hearing device comprises an inertial sensor included in the housing and an orientation evaluator communicatively coupled to the inertial sensor. The orientation evaluator is configured to provide orientation data indicative of a spatial orientation of the housing and/or the wearing position. The hearing device further comprises a walking evaluator configured to provide walking data indicative of a walking activity of the user, and an orientation calibrator configured to provide calibration data depending on the walking data. The calibration data is indicative of a deviation of the spatial orientation of the housing from a reference orientation. The orientation evaluator is configured to provide the orientation data based on the calibration data. The hearing device comprises a controller configured to control an operation of the hearing device based on the orientation data. The hearing device comprises a beamformer, wherein the operation comprises cotnrolling a property of the beamformer. The hearing device comprises a behind-the-ear (BTE) part configured to be worn at a wearing position behind an ear of a user, the BTE part comprising the housing.

In this way, calibration of the orientation evaluator with regard to the reference orientation can be restricted to certain situations in which a walking activity of the user can be assumed which can indicate a certain orientation of the wearing position with respect to the reference orientation. The orientation of the wearing position can thus be kept at least similar during calibration allowing to provide the orientation data in a more reliable way.

Independently, the present disclosure proposes a method of operating a hearing device comprising a housing configured to be worn at a wearing position at an ear of a user. The method comprises providing orientation data indicative of a spatial orientation of the housing and/or the wearing position. The method further comprises providing walking data indicative of a walking activity of the user. The method further comprises providing, depending on the walking data, calibration data indicative of a deviation of the spatial orientation of the housing from a reference orientation. The orientation data is provided based on the calibration data. The method further comprises controlling an operation of the hearing device based on the orientation data, wherein the operation comprises controlling a property of a beamformer included in the hearing device, wherein the hearing device further comprises a behind-the-ear (BTE) part configured to be worn at a wearing position behind an ear of a user, the BTE part comprising said housing.

In some implementations, each hearing device specified above independent from one another can comprise at least one of the subsequently described features. Each of those features can be provided solely or in combination with at least another feature. Each of those features can be correspondingly provided in some implementations of the method of operating the hearing device.

The orientation data may comprise data indicative of a spatial orientation of the housing relative to the wearing position of the housing at the ear and/or data indicative of an orientation of the housing with respect to the reference orientation and/or data indicative of an absolute orientation of the wearing position with respect to the reference orientation. In some implementations, the orientation data indicative of the spatial orientation of the housing comprises orientation data indicative of the spatial orientation of the housing relative to the wearing position. In some implementations, the orientation data indicative of the spatial orientation of the housing comprises the absolute spatial orientation of the housing with respect to the reference orientation, in particular the spatial environment. In some implementations, the orientation data indicative of the spatial orientation of the housing relative to the wearing position and the absolute spatial orientation of the housing with respect to the reference orientation is combined to obtain said orientation data indicative of the spatial orientation of the wearing position.

In some implementations, the orientation calibrator is configured to disregard said deviation from the reference orientation in said calibration data when the walking data is below a threshold of said walking activity. For instance, the walking data can be below the threshold of said walking activity when the walking data indicates that the user is in a resting position. In some implementations, the orientation calibrator is configured to include, in said calibration data, data indicative of said deviation from the reference orientation during a predetermined time. The predetermined time can be selected, for instance, as a time for which the walking data indicates that the user is performing a walking movement. The orientation calibrator can be configured to include, in said calibration data, data indicative of an average value of said deviation averaged over said predetermined time.

In some implementations, the orientation calibrator is configured to estimate the reference orientation in a fixed direction relative to the gravitational force acting on the hearing device. In some implementations, the hearing device comprises a gravitation detector configured to detect the direction of the gravitational force.

The hearing device comprises a controller configured to control an operation of the hearing device based on the orientation data. The controller can be configured to control said operation when the orientation data is indicative of a change of the spatial orientation, in particular the spatial orientation of the housing and/or the spatial orientation of the wearing position. In some implementations, the change of the spatial orientation is selected such that it corresponds to a change of a body orientation of the user between a more upright pose and a more reclined pose of the body of the user.

The hearing device can comprise an output transducer. In some implementations, the operation controlled by the controller comprises changing an amplification of a sound produced by the output transducer. The controller may be communicatively coupled to the output transducer. In some implementations, the operation comprises reducing said amplification when the orientation data is indicative of said change of the spatial orientation corresponding to said more reclined pose of the body of the user.

The hearing device comprises a beamformer. The controller can be communicatively coupled to the beamformer. The operation controlled by the controller comprises controlling a property of the beamformer. In some implementations, the operation comprises steering of a directionality of the beamformer and/or adjusting a beam size, in particular a beam width, of the beamformer.

In some implementations of the device, the adjusting the beam size, in particular a beam width, of the beamformer is controlled when the orientation data is indicative of a head turn of the user. For instance, some of those implementations may be independently applied in a hearing device comprising a housing configured to be worn at a wearing position at an ear of a user, wherein the hearing device comprises an inertial sensor included in the housing and an orientation evaluator communicatively coupled to the inertial sensor, wherein the orientation evaluator is configured to provide orientation data indicative of a spatial orientation of the housing and/or the wearing position. The hearing device further comprises said controller configured to control an operation of the hearing device based on the orientation data. For instance, some of those implementations may be independently applied in a method of operating a hearing device comprising a housing configured to be worn at a wearing position at an ear of a user, the method comprising providing orientation data indicative of a spatial orientation of the housing and/or the wearing position.

In some implementations, the controller is configured to enlarge the beam size, in particular during and/or after the head turn is determined in the orientation data. In some implementations, the controller is configured to control the beamformer to enlarge the beam size for a predetermined time during and/or after the head turn is determined in the orientation data. The controller can be configured to control the beamformer to reduce the beam size when no head turn is determined in the orientation data during and/or after the predetermined time.

In some implementations, the operation is a first operation. The controller can be configured to control a second operation of the hearing device based on the walking data. The second operation can comprise controlling a property of the beamformer. In some implementations, the operation comprises steering of a directionality of the beamformer and/or adjusting a beam size, in particular a beam width, of the beamformer.

The hearing device comprises a BTE part configured to be worn at a wearing position behind an ear of a user. The BTE part comprises said housing.

The inertial sensor is operatively connected to at least one of the orientation evaluator, the orientation calibrator, and the walking evaluator. In some implementations, the inertial sensor is configured to provide movement data indicative of a movement of the housing. In some implementations, the inertial sensor comprises an accelerometer.

In some implementations, the processor is configured with logic providing at least one of the orientation estimator, the orientation calibrator, and the walking estimator. The processor can also be configured with logic providing said controller. Some implementations of the method comprise controlling an operation of the hearing device based on the orientation data.

### BRIEF DESCRIPTION OF THE DRAWINGS

Reference will now be made in detail to embodiments, examples of which are illustrated in the accompanying drawings. The drawings illustrate various embodiments and are a part of the specification. The illustrated embodiments are merely examples and do not limit the scope of the disclosure. Throughout the drawings, identical or similar reference numbers designate identical or similar elements. In the drawings:
- Figs. 1 and 2: schematically illustrate exemplary hearing devices including a sensor unit, a processor, an output transducer, and a microphone, in accordance with some embodiments of the present disclosure;
- Figs. 3 - 5: illustrate exemplary methods of orientation estimation that may be executed by any of the hearing devices illustrated in Figs. 1 and 2;
- Fig. 6: schematically illustrates the hearing device illustrated in Fig. 2 worn at a wearing position at an ear of a user;
- Figs. 7-9: illustrate exemplary methods of orientation estimation that may be executed by any of the hearing devices illustrated in Figs. 1 and 2;
- Figs. 10A, B: schematically illustrate different hearing situations including a hearing system comprising two hearing devices worn at a respective wearing position at the ears of a user;
- Fig. 11: illustrates an exemplary method of orientation estimation that may be executed by any of the hearing devices illustrated in Figs. 1 and 2;
- Fig. 12: illustrates an exemplary signal processing configuration that may be implemented by the hearing devices illustrated in Figs. 1 and 2;
- Fig. 13: illustrates an inertial sensor;
- Fig. 14: illustrates an exemplary signal processing configuration that may be implemented by the hearing devices illustrated in Figs. 1 and 2.

### DETAILED DESCRIPTION OF THE DRAWINGS

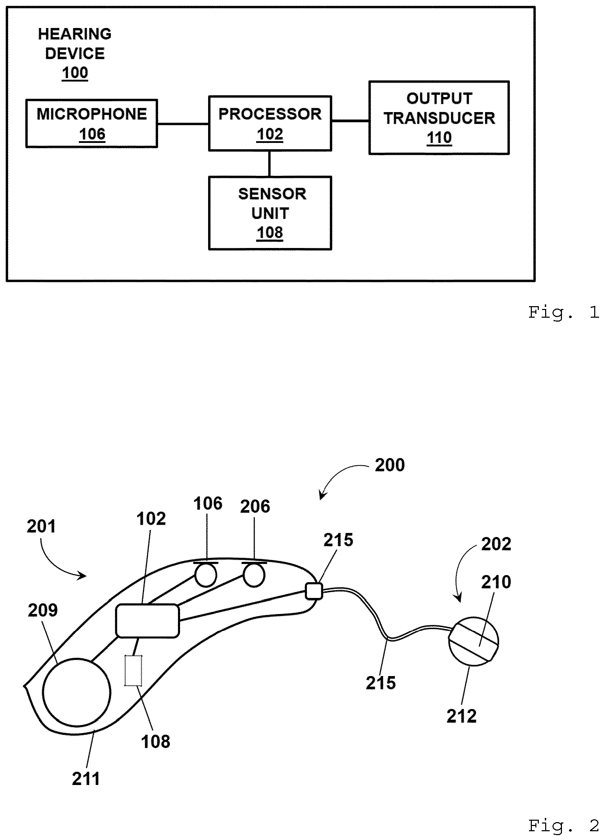

Referring to FIG. 1, a hearing device 100 according to the present disclosure is illustrated. As shown, hearing device 100 includes a processor 102 communicatively coupled to a microphone 106, a sensor unit 108, and an output transducer 110. Hearing device 100 may include additional or alternative components as may serve a particular implementation.

Hearing device 100 may be implemented by any type of hearing device configured to enable or enhance hearing of a user wearing hearing device 100. For example, hearing device 100 may be implemented by a hearing aid configured to provide an amplified version of audio content to a user, an earphone, a cochlear implant system configured to provide electrical stimulation representative of audio content to a user, a sound processor included in a bimodal hearing system configured to provide both amplification and electrical stimulation representative of audio content to a user, or any other suitable hearing prosthesis.

Microphone 106 may be implemented by any suitable audio detection device and is configured to detect a sound presented to a user of hearing device 100. The sound can comprise audio content (e.g., music, speech, noise, etc.) generated by one or more audio sources included in an environment of the user. The sound can also include audio content generated by a voice of the user during an own voice activity, such as a speech by the user. Microphone 106 is configured to output an audio signal comprising information about the sound detected from the environment of the user. Microphone 106 may be included in or communicatively coupled to hearing device 100 in any suitable manner. Output transducer 110 may be implemented by any suitable audio output device, for instance a loudspeaker of a hearing device or an output electrode of a cochlear implant system.

Sensor unit 108 may be implemented by any suitable sensor configured to detect a spatial orientation. To this end, sensor unit 108 comprises an inertial sensor. The inertial sensor can include a motion sensor, for instance an accelerometer, and/or a rotation sensor, for instance a gyroscope. Sensor unit 108 can also comprise a sensor providing information with respect to a deviation of a spatial orientation from a reference orientation. For instance, the direction of a gravitational force may be selected as a reference orientation. Sensor unit 108 may comprise a gravitational sensor configured to detect a direction of gravity. The gravitational sensor can be implemented, for instance, as an accelerometer. Sensor unit 108 can be implemented in a housing of the hearing device configured to be worn at an ear of a user. For instance, the housing may be worn behind the ear and/or at least partially inside the ear canal.

Processor 102 can be configured to access at least one sensor signal generated by vibration sensor 108 and/or the audio signal generated by microphone 106. In this way, processor 102 can be operative to provide an orientation evaluator configured to provide orientation data indicative of a spatial orientation of a housing of hearing device 100. Processor 102 can be operative to provide a walking evaluator configured to provide walking data indicative of a walking activity of the user. Processor 102 can be operative to provide an orientation calibrator configured to provide calibration data indicative of a deviation of the spatial orientation of the housing from a reference orientation. The orientation calibrator can be configured to provide the calibration data depending on the walking data. The orientation evaluator can be configured to provide the orientation data based on the calibration data. For instance, processor 102 can be configured to process orientation data indicative of a spatial orientation of sensor unit 108 and/or a housing including sensor unit 108. Processor 102 may also be configured to process the signal with respect to other signal characteristics which may include said deviation from a reference orientation and/or other motion and/or orientation data such as a movement of the user during a walking activity. These and other operations that may be performed by processor 102 are described in more detail in the description that follows. References to operations performed by hearing device 100 may be understood to be performed by processor 102 of hearing device 100.

FIG. 2 illustrates some exemplary implementations of hearing device 100 as a receiver-in-the-canal (RIC) hearing aid 200, in accordance with some embodiments of the present disclosure. RIC hearing aid 200 comprises a behind-the-ear (BTE) part 201 configured to be worn at an ear at a wearing position behind the ear. Hearing aid 200 further comprises an in-the-ear (ITE) part 202 configured to be worn at the ear at a wearing position at least partially inside an ear canal of the ear. BTE part 201 comprises a housing 211 that can be positioned on the ear at the intended wearing position behind the ear. Housing 211 accommodates processor 102, microphone 106, and sensor unit 108. In the example, an additional microphone 206 is provided in housing 211. Microphones 106, 206 can be included in a microphone array. The microphone array is operationally coupled to processor 102 and may be employed for beamforming. Furthermore, a battery 209 is enclosed by housing 211. ITE part 202 comprises another housing 212 that can be positioned at the ear at an intended wearing position at least partially inside the ear canal. Output transducer 110 is implemented in hearing aid 200 as a receiver 210. Receiver 210 is accommodated in housing 212 of ITE part 202. BTE part 201 and ITE part 202 are interconnected by a cable 215. Receiver 210 is operationally coupled to processor 102 via cable 215 and a cable connector 216 provided at housing 211 of BTE part 201. Alternatively, a wireless coupling between processor 102 and receiver 210 is conceivable.

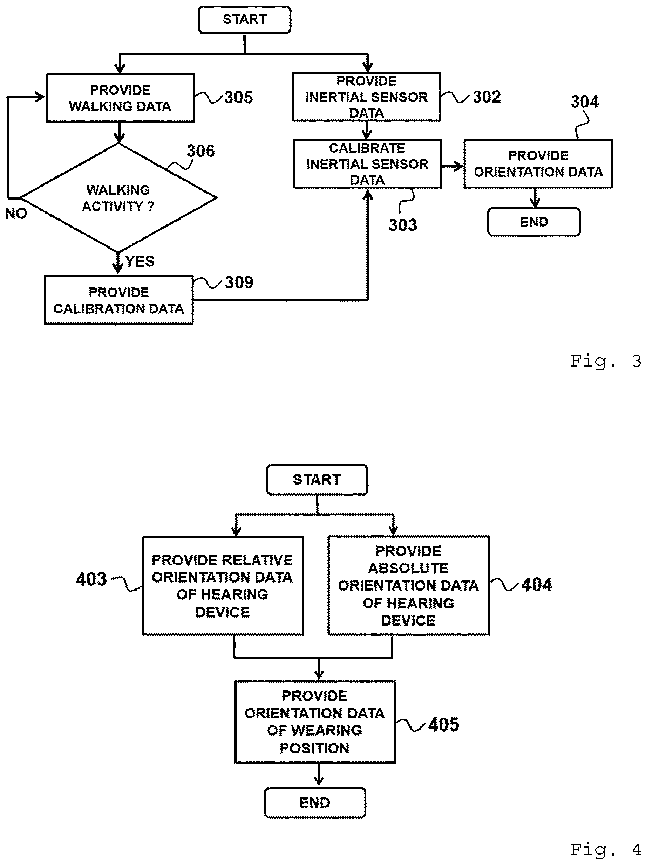

FIGS. 3 - 5, 7 - 9, and 11 illustrate exemplary methods of operating a hearing device.

FIG. 3 illustrates a method of operating a hearing device for providing orientation data. As shown, inertial sensor data is provided in operation 302. The inertial sensor data can be any data comprising information about a spatial orientation of a housing of a hearing device during wearing at an ear of a user. For instance, the inertial sensor data can comprise data relative to a movement and/or a rotation of the housing. For instance, the inertial sensor of sensor unit 108 can provide the inertial sensor data. In operation 303, the inertial sensor data is calibrated based on calibration data. The calibration data can be indicative of a deviation of the spatial orientation of the housing from a reference orientation, as further described below. In operation 304, the orientation data based on the calibration data is provided. The orientation data can comprise data indicative of a spatial orientation of the housing relative to the wearing position of the housing at the ear. The orientation data can comprise data indicative of an absolute orientation of the housing relative to the ambient environment of the user. The orientation data can comprise data indicative of an absolute orientation of the wearing position of the housing at the ear. For instance, the orientation data can be provided by an orientation evaluator operated by processor 102.

In operation 305, walking data is provided. The walking data is indicative of a walking activity of the user. The walking data can comprise data relative to a movement and/or a rotation of the user wearing the hearing device. For instance, the walking data can comprise the inertial sensor data provided in operation 302. Alternatively or additionally, the walking data can comprise other data relative to the walking activity of the user, in particular data provided from another sensor. For instance, the additional sensor may be included in sensor unit 108 or provided externally from hearing device 100. The walking data can be provided by a walking estimator. For instance, the walking estimator can be operated by processor 102. The walking estimator can be operatively coupled to the inertial sensor and/or said other sensor. In operation 306, a decision is performed depending on the walking data. In a case in which the walking data is not indicating a walking activity of the user, operation 305 is repeated. In a case in which the walking data is indicating a walking activity of the user, calibration data is provided in operation 309. The decision in orientation operation 306 can be based on whether the walking data is below a threshold of the walking activity. For instance, the threshold can be selected such that the walking data is below threshold for a walking data indicative of the user in a resting position, e.g. when the user sits, lies, and/or stands still. In such a situation below threshold, the reference orientation may be disregarded in said calibration data, in particular not included in the calibration data. Thus, the information in the calibration data can be restricted to the orientation deviations from the reference orientation for the walking data above threshold.

The calibration data provided in operation 309 depending on the walking data can be indicative of a deviation of the spatial orientation of the housing from a reference orientation. In particular, the reference orientation may be estimated in a fixed direction relative to the gravitational force acting on the hearing device. Operation 309 can comprise providing reference orientation data indicative of a direction of the reference orientation. Operation 309 can comprise providing deviation data indicative of a deviation of an actual orientation, in particular a momentary orientation, from the reference orientation. For instance, a reference orientation sensor can be employed to provide the reference orientation data. The reference orientation sensor can comprise an inertial sensor, in particular an accelerometer. For instance, the reference orientation data and/or deviation data can comprise the inertial sensor data provided in operation 302. Alternatively or additionally, the reference orientation data and/or deviation data can comprise other data relative to the deviation of the spatial orientation of the housing from the reference orientation, in particular data provided from another sensor. For instance, the additional sensor may be included in sensor unit 108 or provided externally from hearing device 100. Operations 305, 306, 309 can be executed, for instance, in parallel to operations 302, 303, 304 and/or before and/or after operations 302, 303, 304. The calibration data provided in operation 309 is then employed in operation 303 to calibrate the inertial sensor data. For instance, the orientation evaluator operated by processor 102 can be configured to provide the orientation data in operation 304 based on the calibration data.

FIG. 4 illustrates a method of operating a hearing device for providing orientation data. In operation 403, relative orientation data of the hearing device is provided. The relative orientation data can comprise data indicative of a spatial orientation of the housing relative to the wearing position of the housing at the ear. The relative orientation data can be determined by employing the calibration data provided in operation 309 indicative of a deviation of the spatial orientation of the housing from a reference orientation, and the inertial sensor data provided in operation 302 indicative of the spatial orientation of the housing during wearing at an ear of a user, in particular with respect to the reference orientation. For instance, the relative orientation data can be determined by a difference of the inertial sensor data provided in operation 302 and the calibration data provided in operation 309. In operation 404, absolute orientation data of the hearing device is provided. The absolute orientation data can comprise data indicative of a spatial orientation of the housing with respect to the spatial surroundings of the user. For instance, the inertial sensor data provided in operation 302 indicative of the spatial orientation of the housing can be employed to provide the absolute orientation data.

In operation 405, orientation data of the wearing position of the hearing device is provided. The orientation data of the wearing position can comprise, for instance, orientation data of the ear at which the housing is worn. The orientation data of the wearing position can thus be independent from the orientation of the housing of the hearing device. The orientation data of the wearing position can comprise absolute orientation data of the wearing position indicative of a spatial orientation of the wearing position with respect to the spatial surroundings of the user. For instance, the absolute orientation data can be indicative of the orientation of the wearing position with respect to the reference orientation. The orientation data of the wearing position can be determined, for instance, by employing the relative orientation data provided in operation 403 comprising data indicative of a spatial orientation of the housing relative to the wearing position, and the absolute orientation data provided in operation 404 comprising data indicative of a spatial orientation of the housing with respect to the spatial surroundings of the user. For instance, the orientation data of the wearing position can be determined by a difference of the relative orientation data provided in operation 403 and the absolute orientation data provided in operation 404.

The method can be operated during and/or after at least one of operations 303, 304 described above. For instance, the method can be executed by the orientation evaluator operated by processor 102. In some implementations, at least one of operations 403, 404, 405 is performed, in particular in operation 304 described above. In some implementations, only operation 403 or operation 404 or operation 405 is performed. In some implementations, operations 403, 404, 405 are performed in a different order.

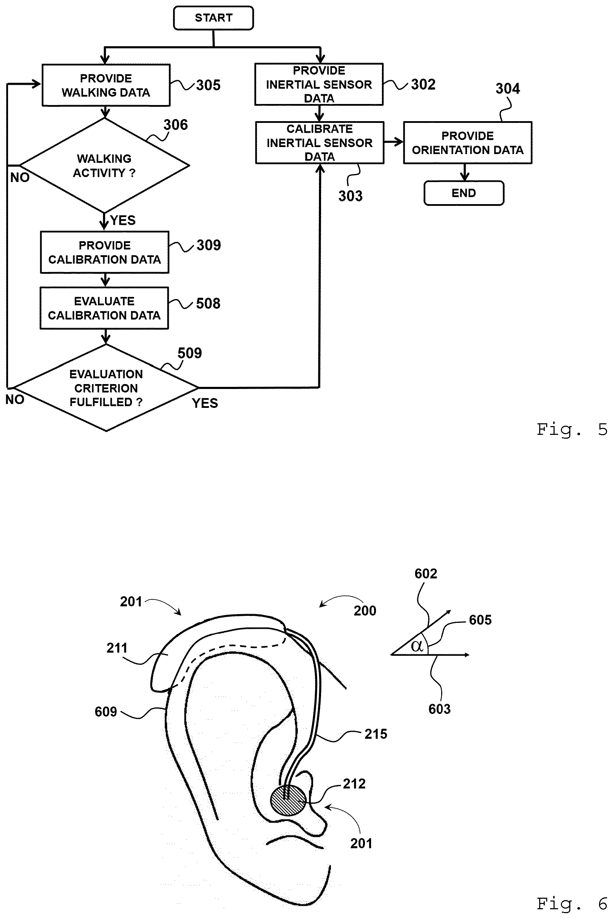

FIG. 5 illustrates a method of operating a hearing device for providing orientation data. In operation 508, the calibration data provided in operation 309 is evaluated with respect to an evaluation criterion. In operation 509, a decision is performed depending on the evaluation criterion. In a case in which the evaluation criterion is not fulfilled, operation 305 is repeated. In a case in which the evaluation criterion is fulfilled, the evaluated calibration data is provided for the calibration performed in operation 303.

The evaluation can be performed with respect to a predetermined time such that the evaluated calibration data is indicative of said deviation from the reference orientation during the predetermined time. The evaluation criterion can comprise a duration of the walking activity exceeding the predetermined time. In particular, the evaluated calibration data can be indicative of an average value of said deviation averaged over said predetermined time. For instance, a long term mean of the calibration data provided in operation 309 may be provided in such a way. The evaluation can be performed with respect to a predetermined threshold of a parameter, in particular a probabilistic parameter such as a standard deviation from a mean value of said deviation from the reference orientation and/or a variance of said deviation from the reference orientation, such that the evaluated calibration data is indicative of said deviation from the reference orientation in consideration of the predetermined threshold of the parameter. For instance, the evaluated calibration data can thus be provided below a predetermined threshold of a standard deviation and/or a variance. The evaluation criterion can comprise a value determined for said parameter during the walking activity above or below the predetermined parameter threshold, for instance below a predetermined standard deviation threshold and/or variance threshold.

FIG. 6 illustrates hearing device 200 illustrated in Fig. 2 worn at a wearing position at an ear of a user 609. Housing 211 of BTE part 201 is positioned at the wearing position at ear 609 behind the ear. Variances of the wearing position of housing 211 may exist when applying housing at the wearing position at different times. Those variances can result in different orientations of housing with respect to the ambient environment and/or a reference orientation such as a gravitational force acting on housing 211. An axis of orientation 602 of housing 211 is defined by an axis having a fixed position relative to the housing. An axis of a reference orientation 603 is defined by an axis having a fixed position with respect to the ambient environment, for instance with respect to the earth surface and/or the direction of gravity. A deviation angle 605 of the orientation of housing 311 from the reference orientation is defined between housing orientation axis 602 and reference orientation axis 603.

Sensor unit 108 including an inertial sensor may be employed to determine an orientation. Sensor unit 108 is implemented in housing 211. The orientation 602 of housing 211 with respect to reference orientation 603 may thus be determined by sensor unit 108. Moreover, if the orientation of housing 211 relative to the wearing position at ear 609 would be additionally known, the orientation of the wearing position itself, in particular of ear 609, with respect to reference orientation 603 could be derived from the orientation 602 of housing 211 with respect to reference orientation 603. But the orientation of housing 211 relative to the wearing position at ear 609 can vary, for instance, at different wearing times resulting in varying deviation angles 605. Thus, neither the orientation of housing 211 relative to the wearing position at ear 609 nor the orientation of the wearing position is known from the orientation information provided by the inertial sensor included in sensor unit 108. The methods illustrated in FIGS. 3-5, however, can be employed to estimate both, the orientation of housing 211 relative to the wearing position at ear 609 and the orientation of the wearing position.

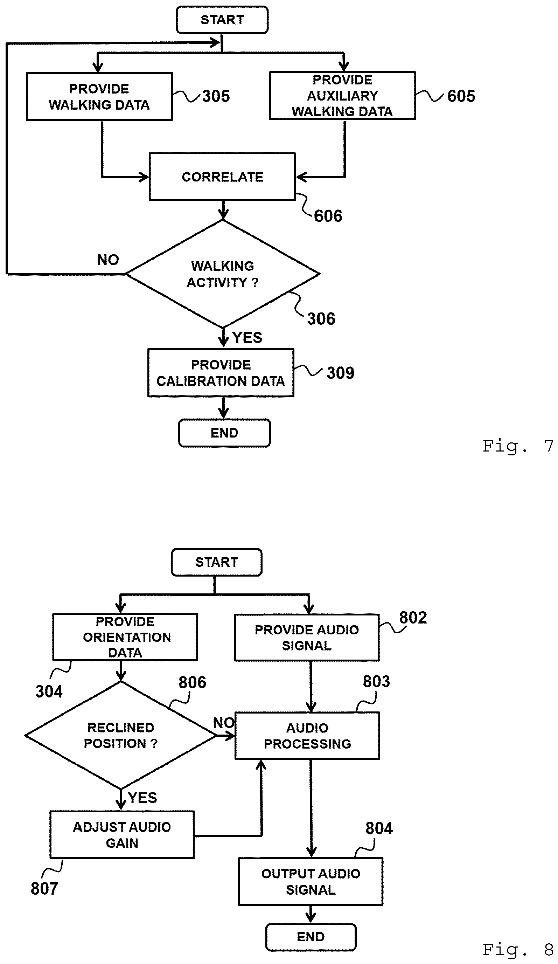

FIG. 7 illustrates a method of operating a hearing system for providing orientation data. In operation 705, auxiliary walking data is provided. The auxiliary walking data can be provided in a corresponding way as described above in conjunction with operation 305. An auxiliary sensor, in particular an auxiliary inertial sensor, may be employed to provide auxiliary data relative to a movement and/or a rotation of the user wearing the hearing device. In operation 706, the walking data provided in operation 305 is correlated with the auxiliary walking data provided in operation 705. The correlated walking data is then employed in decision operation 306. In some implementations, the method is performed in the place of operations 305, 306, 309 in the method illustrated in FIG. 3 and/or in the method illustrated FIG. 4.

The hearing system can be a binaural hearing system comprising a first hearing device configured to be worn at a first ear, and a second hearing device configured to be worn at a second ear. For instance, each of the first and second hearing device may be provided by hearing device 100 illustrated in FIG. 1 or hearing aid 200 illustrated in FIG. 2. The first hearing device may comprise a first housing in which a first sensor unit is included. The second hearing device may comprise a second housing in which a second sensor unit is included. The first sensor unit may include said sensor and the second sensor unit may include said auxiliary sensor. The walking estimator can be operatively coupled to the first sensor unit and the second sensor unit, for instance to an inertial sensor and/or another sensor included in the respective sensor unit. In some implementations, the orientation calibrator can be operatively coupled to the first sensor unit and the second sensor unit. The deviation from the reference orientation based on sensor data of each sensor unit can then also be correlated. In some implementations, the orientation evaluator can be operatively coupled to the first sensor unit and the second sensor unit. The orientation data based on sensor data of each sensor units can then also be correlated.

FIG. 8 illustrates a method of operating a hearing device for providing orientation data. In operation 802, an audio signal is provided. For instance, the audio signal can be provided by microphone 106. The audio signal is then processed in operation 803. For instance, the signal processing can be operated by processor 102. The signal processing can comprise, for instance, providing an amplification, in particular a gain, of the audio signal. In operation 804, the audio signal is outputted, for instance by output transducer 110.

Simultaneously, after providing the orientation data in operation 304, a decision 806 is performed. The decision depends on whether the user is in a reclined body pose. An indication of such a body pose can be provided by the orientation data. In particular, the orientation data can comprise information about the orientation of the wearing position of the hearing device at the ear, as determined, for instance, in operation 403. The reclined body pose can be an indicator that the user is lying down on a bed or leaning back on a sofa. In such a situation there is a risk of increased feedback in the hearing device. For instance, when BTE part 201 of hearing device 200, as illustrated in FIG. 6, approaches a bed surface, backrest, pillow and/or the like in the reclined body pose, this can provoke increasing feedback of microphones 106, 206 included in BTE part 201. The feedback risk can be reduced by reducing the amplification of the audio signal processed in operation 803 by a certain amount. Thus, if a change of the user's body pose from an upright pose to a reclined pose is estimated in operation 806, the audio gain is adjusted in operation 807 before the audio signal is output in operation 804. The audio signal gain can then be reduced by a certain amount. In a reverse situation, if a change of the user's body pose from a reclined pose to an upright pose is estimated in operation 806, the audio gain can also be adjusted in operation 807 before the audio signal is output in operation 804. The audio signal gain can then be increased to the customary amount. If no such a change of the body pose is found in operation 806, the audio processing in operation 803 is continued in operation 803 in an unaffected way.

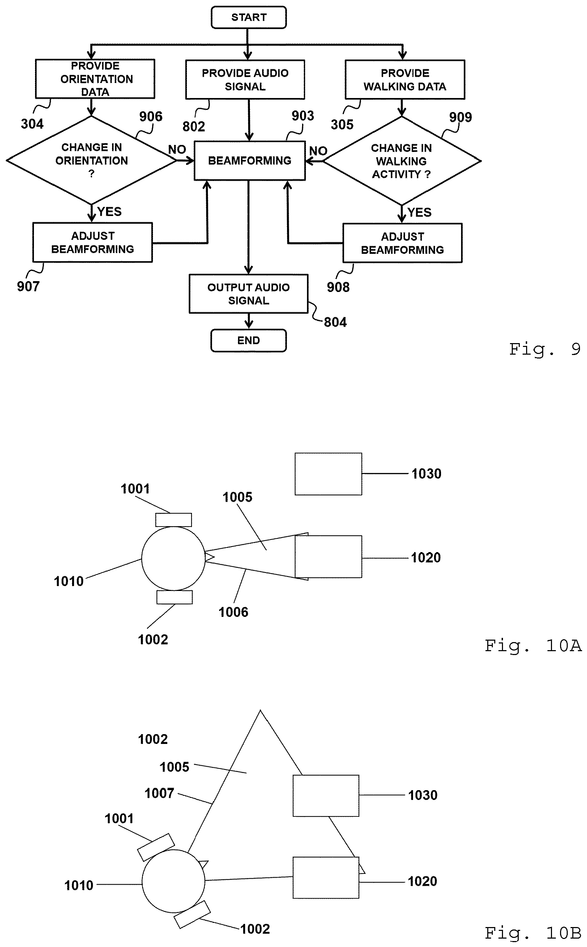

FIG. 9 illustrates a method of operating a hearing device for providing orientation data. In operation 903, beamforming is performed in the audio signal provided in operation 802. For instance, multiple audio signals can be provided in operation 802 by a microphone array including microphone 106 which can be processed by a beamformer. The beamformer can be operated, for instance, by processor 102. The beamformed audio signal is outputted in operation 804.

In some implementations, a decision is performed in operation 906 based on the orientation data provided in operation 304. If the orientation data is indicative for a change in orientation, the beamforming is adjusted in operation 907. For instance, a directionality of the beamformed audio signal can be changed. For instance, a beam size of the beamformed audio signal can be changed. The change of orientation can comprise, for instance, an orientation change of the wearing position of the hearing device as determined in operation 405. This can indicate a head movement of the user, such as a head turn. If the orientation data is not indicative for a change in orientation, the beamforming is maintained in operation 903 in an unaffected manner by operation 906.

In some implementations, a decision is performed in operation 909 based on the walking data provided in operation 305. If the walking data is indicative for a change in the walking activity, the beamforming is adjusted in operation 908. For instance, a directionality of the beamformed audio signal can be changed. For instance, a beam size of the beamformed audio signal can be changed. The change of the walking activity can comprise, for instance, an different walking speed determined in the walking data and/or a change from a resting situation to a walking situation. If the orientation data is not indicative for a change in the walking activity, the beamforming is maintained in operation 903 in an unaffected manner by operation 906.

In some implementations of the method, operations 909, 908 based on the walking data and operations 906, 907 based on the orientation data are performed. In some implementations of the method, operations 909, 908 based on the walking data are performed, wherein operations 906, 907 are omitted. In some implementations of the method, operations 906, 907 based on the orientation data are performed, wherein operations 909, 908 are omitted.

FIGS. 10A and 10B schematically illustrate different hearing situations involving a user 1010 and two communication partners 1020, 1030. User 1010 is wearing a binaural hearing system comprising two hearing devices 1001, 1002 worn at a respective wearing position at the ears of a user. Each of hearing devices 1001, 1002 can be implemented, for instance, by hearing device 100 shown in FIG. 1 and/or hearing device 200 shown in Fig. 2. Hearing devices 1001, 1002 are configured to provide binaural beamforming of an audio signal. The beamformed audio signal can be based on multiple audio signals detected by a microphone array including, for instance, microphone 106. The beamformed audio signal can be outputted, for instance, by output transducer 110. The beamforming may be provided by a beamformer operated, for instance, by processor 102. An angular range 1005 of the beamforming, as perceived by user 1010, is schematically illustrated.

In the situation illustrated in FIG. 10A, user 1010 faces first communication partner 1020 with whom he is involved in a face-to-face conversation. In this situation, angular range 1005 defines a rather narrow beam 1006 focused on the sound produced by first communication partner 1020. Thus, the user can mainly hear the sound produced during the bilateral conversation with first communication partner 1020. In the situation illustrated in FIG. 10B, user 1010 has turned his head such that he faces second communication partner 1030. Here the user is involved in a group conversation including both communication partners 1020, 1030. In this situation, angular range 1005 defines a rather wide beam 1007 encompassing the sound produced by both communication partners 1020, 1030. Thus, the user can perceive the sound produced multilaterally by the participants 1010, 1020, 1030.

Turning the head of the user, as illustrated in FIGS. 10A, 10B can give an indication of the user's intention to switch from a bilateral conversation to a group conversation, in particular such that the user can perceive sound of additional conversation partners. In this case, an automatic adjustment of the beamforming, in particular from narrow beam 1006 to wider beam 1007, would be desirable. The orientation data provided in operation 304 can contain information about a head turn and/or other head movement of the user. For instance, a change of the orientation data of the wearing position as provided in operation 405 can indicate the head turn and/or other head movement. The beamforming can thus be adjusted accordingly in operation 907. In this way, the user may not loose information coming from the conversation with his conversation partner during and/or after the head turn. This can avoid that the user needs to put additional effort in following group conversations with a beamformed signal, for instance when beamforming is required for an improved signal-to-noise ratio.

In some implementations, an angle of the head turn performed by the user can be determined based on the orientation data. An amount of adjustment of the beamforming in operation 907 can then be variably selected in operation 907. For instance, when the user only slightly turns his head such that the turning angle would be small, the beam size may be only increased to a slightly wider beam size. When the user turns his head by a larger amount such that the turning angle would be large, the beam size may be increased to a beam size with a larger width.

After a certain time interval, when no further head turns of the user are determined in the orientation data, an automatic re-adjustment of the beamforming from wider beam 1007 to narrow beam 1006 may be performed in operation 907. In particular, a lack of further head turns within the time interval can indicate that the user wants to focus again on a single conversation. Indications that the user does not want to include additional partners in his conversation with a single person can also be, for instance, a continuous back and forth movement in the same direction, for instance in the direction of the single person the user intends to speak. Such a movement may be also detected by sensor unit 108, in particular by the inertial sensor. When such a movement is detected, the automatic re-adjustment of the beamforming may also be performed. The adjustment of the beamforming can also be selected to depend on further criteria. For instance, own voice detection may be applied to identify the user's intention to communicate. The adjustment of the beamforming can then also depend on the detected own voice. For instance, an environmental classification may be employed to identify the user's intention to communicate and/or the user's intention to communicate with a single person or a group of people. The environment can be classified with respect to different types of acoustic scenes including, for instance, calm scenes, a speech in noise, a speech in loud noise, and/or the like. The adjustment of the beamforming can then also depend on the identified acoustic scene.

In some implementations of the present disclosure, the above described operations illustrated in FIG. 10A and FIG. 10B may be performed in a hearing device comprising a walking evaluator and a orientation calibrator, in particular in the above described way. In some implementations of the present disclosure, the above described operations illustrated in FIG. 10A and FIG. 10B may be performed in a hearing device in which a walking evaluator and a orientation calibrator, as described above, can be omitted. In such a hearing device, the orientation data may be provided by another orientation evaluator known in the art and/or another method of obtaining the orientation data known in the art. In particular, the above described operations illustrated in FIG. 10A and FIG. 10B may be independently applied in a hearing device comprising a housing configured to be worn at a wearing position at an ear of a user, wherein the hearing device comprises an inertial sensor included in the housing and an orientation evaluator communicatively coupled to the inertial sensor, wherein the orientation evaluator is configured to provide orientation data indicative of a spatial orientation of the housing and/or the wearing position. In some implementations, the hearing device comprises a controller operatively coupled to the orientation evaluator. In some implementations, the controller is configured to enlarge the beam size, in particular during and/or after the head turn is determined in the orientation data. In some implementations, the controller is configured to control the beamformer to enlarge the beam size for a predetermined time during and/or after the head turn is determined in the orientation data. The controller can be configured to control the beamformer to reduce the beam size when no head turn is determined in the orientation data during and/or after the predetermined time.

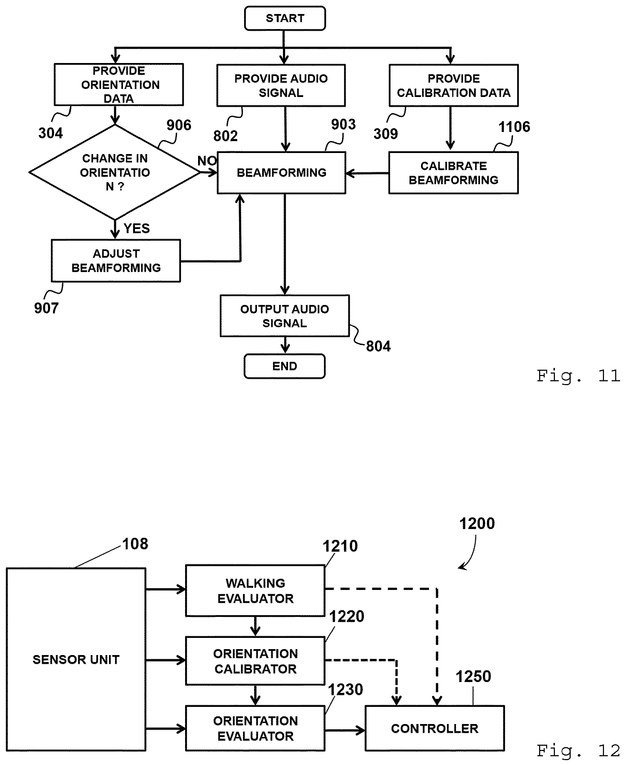

FIG. 11 illustrates a method of operating a hearing device for providing orientation data. In operation 1106, after providing the calibration data in operation 309, the beamforming is calibrated. The calibration data can include the deviation data of the housing orientation from the reference orientation. In this way, the beamforming can be calibrated with respect to the reference orientation, for instance the direction of gravity. The calibration operation 1106 may be performed before and/or during the beamforming in operation 903. In some implementations, the calibration of the beamformer with respect to the reference orientation is provided in a rather slow manner, in particular by an adaptive level matching. This may allow to avoid an undesired short-term adjustment of the beamforming, for instance in situations in which the user shortly looks up, such that only longer lasting positioning effects could be compensated for. In some implementations of the method, a beamforming adjustment according to operations 304, 906 and 1106 can be performed simultaneously and/or after the beamforming calibration in operations 309, 1106. In some implementations of the method, a beamforming adjustment according to operations 304, 906 and 1106 can be omitted.

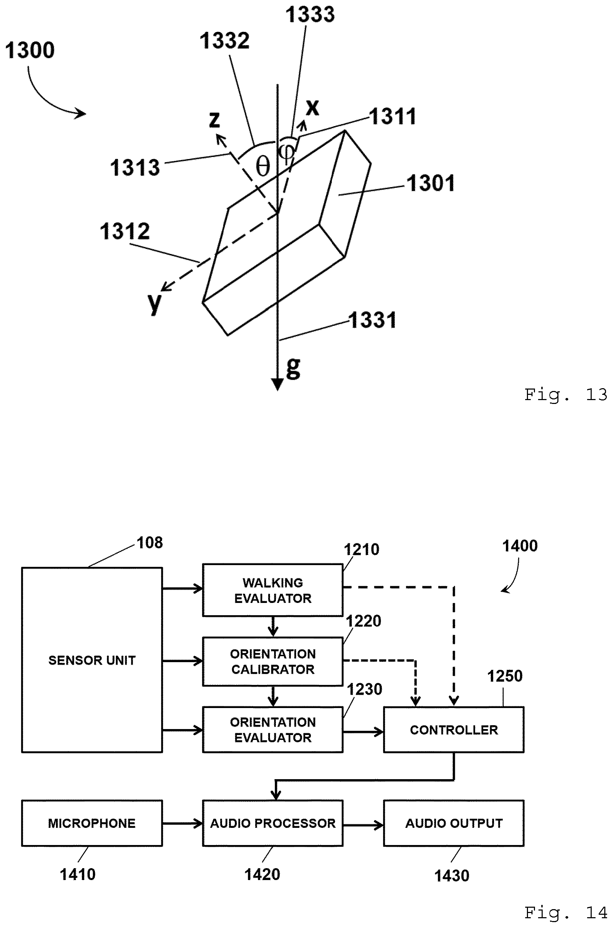

FIGS. 12 and 14 illustrate exemplary signal processing configurations of a hearing device. Some of the illustrated functional components may be operated by processor 102, for instance in a signal processing routine, algorithm, program and/or the like. Other illustrated functional components may be operatively coupled to processor 102, for instance to provide and/or modify a signal processed by processor 102. Some embodiments may be implemented in a hearing device comprising additional constituent parts, for instance an additional microphone and/or beamformer. Some embodiments may be implemented in a hearing system comprising two hearing devices in a binaural configuration.

FIG. 12 illustrates an exemplary signal processing configuration 1200 for a signal processing of a sensor signal provided by sensor unit 108. As shown, signal processing configuration 1200 comprises a walking evaluator 1210. Walking evaluator 1210 can be configured to provide walking data indicative of a walking activity of the user. For instance, walking evaluator 1210 can be configured to execute operations 305, 305 described above. Signal processing configuration 1200 further comprises an orientation calibrator 1220 operatively coupled to walking evaluator 1210. Orientation calibrator 1220 can be configured to provide calibration data indicative of a deviation of the spatial orientation of a housing of the hearing device from a reference orientation. Orientation calibrator 1220 can be configured to provide the calibration data depending on the walking data provided by walking evaluator 1210. For instance, orientation calibrator 1220 can be configured to execute operation 309 and/or operations 508, 509 described above. Signal processing configuration 1200 further comprises an orientation evaluator 1230 operatively coupled to orientation calibrator 1220. Orientation evaluator 1230 can be configured to provide orientation data indicative of a spatial orientation of at least one of the housing and the wearing position. Orientation evaluator 1230 can be configured to provide the orientation data based on the calibration data provided by orientation calibrator 1220. For instance, orientation evaluator 1230 can be configured to execute operations 303, 304 and/or at least one of operations 403, 404, 405 described above.

Signal processing configuration 1200 further comprises a controller 1250 operatively coupled to orientation evaluator 1230. Controller 1250 can be configured to control an operation of the hearing device based on the orientation data provided by orientation evaluator 1230. In some implementations, as illustrated in Fig. 11 by dashed lines, controller 1250 is operatively coupled to walking evaluator 1230. Controller 1250 can be configured to control an operation of the hearing device based on the walking data provided by walking evaluator 1210. In some implementations, as illustrated in Fig. 11 by further dashed lines, controller 1250 is operatively coupled to orientation calibrator 1220. Controller 1250 can be configured to control an operation of the hearing device based on the calibration data provided by walking evaluator 1220. For instance, controller 1250 can be configured to execute operation 807 and/or operation 907 and/or operation 908 and/or operation 1106 described above.

FIG. 13 illustrates an inertial sensor 1300. Inertial sensor 1300 is provided by an accelerometer 1301 which may be configured to detect accelerations in one, two, or three distinct spatial directions. In the illustrated example, accelerometer 1301 is configured to detect accelerations in three spatial directions including an x-direction 1311, a y-direction 1312, and a z-direction 1313. When implemented in a hearing device housing worn at a user's ear, accelerometer 109 is thus configured to provide a respective acceleration signal indicative of a movement in each of spatial directions 1311 - 1312. The acceleration signals thus contain information about the momentary spatial orientation of the housing. Accelerometer 1301 is further configured to detect a direction of an axis 1331 pointing in a direction of a gravitational force. Axis 1331 of gravitational force can be employed as an axis of a reference orientation. In this way, accelerometer 1301 is configured to detect a deviation angle 1332 of z-direction 1313 with respect to reference orientation axis 1331 and/or to detect a deviation angle 1333 of x-direction 1311 with respect to reference orientation axis 1331. The acceleration signals thus also contain information about the spatial orientation of the housing with respect reference orientation axis 1331. The acceleration signals can further be exploited to provide information about a walking activity of the user. In particular, the accelerometer signal can be exploited with respect to a mean number of crossings of a movement in at least one of spatial directions 1311 - 1312. Such a crossing can indicate, for instance, subsequent steps performed by the user during walking. For instance, an evaluation of mean crossings in y-direction 1312 can indicate if the user is walking or in a resting position and/or how fast the user is walking. Sensor unit 108 may comprise or consist of inertial sensor 1300.

FIG. 14 illustrates an exemplary signal processing configuration 1400 for a signal processing of a sensor signal provided by sensor unit 108. Signal processing configuration further comprises a microphone 1410. Microphone 1410 can be provided, for instance, by microphone 106 and/or microphone 206 described above. Microphone 1410 can also be provided by a microphone array. Microphone 1410 can be configured to provide an audio signal. As shown, signal processing configuration 1400 further comprises an audio processor 1420 configured to process the audio signal. In particular, audio processor 1420 can be configured to perform operation 803 and/or operation 903 described above. Signal processing configuration further comprises an output 1430 for the audio signal, for instance an acoustic transducer. Controller 1250 is operatively coupled to audio processor 1420. Thus, the audio processing can be controlled by controller 1250, in particular by executing operation 807 and/or operation 907 and/or operation 908 and/or operation 1106 described above.

While the principles of the disclosure have been described above in connection with specific devices and methods, it is to be clearly understood that this description is made only by way of example and not as limitation on the scope of the invention. The above described preferred embodiments are intended to illustrate the principles of the invention, but not to limit the scope of the invention. Various other embodiments and modifications to those preferred embodiments may be made by those skilled in the art without departing from the scope of the present invention that is solely defined by the claims.

## Claims

1. A hearing device comprising a housing (211, 212) configured to be worn at a wearing position at an ear of a user, the hearing device comprising
- an inertial sensor (1300) included in the housing (211, 212);
- an orientation evaluator (1230) communicatively coupled to the inertial sensor (1301), the orientation evaluator (1230) configured to provide orientation data indicative of a spatial orientation of the housing (211, 212) and/or the wearing position;
- a walking evaluator (1210) configured to provide walking data indicative of a walking activity of the user;
- an orientation calibrator (1220) configured to provide calibration data indicative of a deviation of the spatial orientation of the housing (211, 212) from a reference orientation, the orientation calibrator (1220) configured to provide the calibration data depending on the walking data, wherein the orientation evaluator (1230) is configured to provide the orientation data based on the calibration data; and
- a controller (1250) configured to control an operation of the hearing device based on the orientation data,
- a beamformer, wherein the operation comprises controlling a property of the beamformer; and
- a behind-the-ear part (201) configured to be worn at a wearing position behind an ear of a user, the behind-the-ear part comprising said housing.

2. The device according to claim 1, wherein the orientation calibrator (1220) is configured to disregard said deviation from the reference orientation in said calibration data when the walking data is below a threshold of said walking activity.

3. The device according to claim 1 or 2, wherein the orientation calibrator (1220) is configured to include, in said calibration data, data indicative of said deviation from the reference orientation during a predetermined time.

4. The device according to claim 3, wherein the orientation calibrator (1220) is configured to include, in said calibration data, data indicative of an average value of said deviation averaged over said predetermined time.

5. The device according to any of the preceding claims, wherein the orientation calibrator (1220) is configured to estimate said reference orientation in a fixed direction relative to a gravitational force acting on the hearing device.

6. The device according to claim 5, wherein the controller (1250) is configured to control said operation when the orientation data is indicative of a change of the spatial orientation of the housing and/or the wearing position.

7. The device according to claim 6, wherein said change of the spatial orientation is selected such that it corresponds to a change of a body orientation of the user between a more upright pose and a more reclined pose of the body of the user.

8. The device according to any of the preceding claims, further comprising an output transducer (110, 210, 1430), wherein the operation controlled by the controller (1250) comprises changing an amplification of a sound produced by the output transducer (110, 210, 1430).

9. The device according to claims 7 and 8, wherein the operation comprises reducing said amplification when the orientation data is indicative of said change of the spatial orientation corresponding to said more reclined pose of the body of the user.

10. The device according to any of the preceding claims, wherein the operation is a first operation, wherein the controller (1250) is configured to control a second operation of the hearing device based on the walking data.

11. The device according to any of the preceding claims, wherein the inertial sensor (1300) comprises an accelerometer (1301).

12. A method of operating a hearing device comprising a housing (211, 212) configured to be worn at a wearing position at an ear of a user, the method comprising
- providing orientation data indicative of a spatial orientation of the housing (211, 212) and/or the wearing position,
- providing walking data indicative of a walking activity of the user;
- providing, depending on the walking data, calibration data indicative of a deviation of the spatial orientation of the housing (211, 212) from a reference orientation, wherein the orientation data is provided based on the calibration data; and
- controlling an operation of the hearing device based on the orientation data, wherein the operation comprises controlling a property of a beamformer included in the hearing device, wherein the hearing device further comprises a behind-the-ear part (201) configured to be worn at a wearing position behind an ear of a user, the behind-the-ear part comprising said housing.

## Patentansprüche

1. Hörgerät, umfassend ein Gehäuse (211, 212), das ausgestaltet ist, an einer Trageposition an einem Ohr eines Anwenders getragen zu werden, wobei das Hörgerät umfasst:
- einen Trägheitssensor (1300), der in das Gehäuse (211, 212) eingeschlossen ist;
- einen Orientierungsevaluierer (1230), der kommunikativ an den Trägheitssensor (1301) gekoppelt ist, wobei der Orientierungsevaluierer (1230) ausgestaltet ist, um Orientierungsdaten bereitzustellen, die eine räumliche Orientierung des Gehäuses (211, 212) und/oder die Trageposition angeben;
- einen Geh-Evaluierer (1210), der ausgestaltet ist, um Geh-Daten bereitzustellen, die eine Geh-Aktivität des Anwenders angeben;
- einen Orientierungskalibrator (1220), der ausgestaltet ist, um Kalibrierungsdaten bereitzustellen, die eine Abweichung der räumlichen Orientierung des Gehäuses (211, 212) von einer Referenzorientierung angeben, wobei der Orientierungskalibrator (1220) ausgestaltet ist, um die Kalibrierungsdaten in Abhängigkeit von den Geh-Daten bereitzustellen, wobei der Orientierungsevaluierer (1230) ausgestaltet ist, um die Orientierungsdaten basierend auf den Kalibrierungsdaten bereitzustellen; und
- eine Steuerung (1250), die ausgestaltet ist, um einen Betrieb des Hörgeräts basierend auf den Orientierungsdaten zu steuern,
- einen Strahlformer, wobei der Betrieb Steuern einer Eigenschaft des Strahlformers umfasst; und
- ein Hinter-dem-Ohr-Teil (201), das ausgestaltet ist, um an einer Trageposition hinter dem Ohr eines Anwenders getragen zu werden, wobei das Hinter-dem-Ohr-Teil das Gehäuse umfasst.

2. Gerät nach Anspruch 1, wobei der Orientierungskalibrator (1220) ausgestaltet ist, um die Abweichung von der Referenzorientierung in den Kalibrierungsdaten nicht zu beachten, wenn die Geh-Daten unter einem Schwellenwert der Geh-Aktivität liegen.

3. Gerät nach Anspruch 1 oder 2, wobei der Orientierungskalibrator (1220) ausgestaltet ist, um in die Kalibrierungsdaten Daten einzuschließen, die die Abweichung von der Referenzorientierung während einer vorbestimmten Zeit angeben.

4. Gerät nach Anspruch 3, wobei der Orientierungskalibrator (1220) ausgestaltet ist, um in die Kalibrierungsdaten Daten einzuschließen, die einen Durchschnittswert der Abweichung angeben, gemittelt über die vorbestimmte Zeit.

5. Gerät nach einem der vorhergehenden Ansprüche, wobei der Orientierungskalibrator (1220) ausgestaltet ist, um die Referenzorientierung in einer festen Richtung relativ zu einer Schwerkraft zu schätzen, die auf das Hörgerät einwirkt.

6. Gerät nach Anspruch 5, wobei die Steuerung (1250) ausgestaltet ist, um den Betrieb zu steuern, wenn die Orientierungsdaten eine Änderung in der räumlichen Orientierung des Gehäuses und/oder der Trageposition angeben.

7. Gerät nach Anspruch 6, wobei die Änderung der räumlichen Orientierung so ausgewählt wird, dass sie einer Änderung einer Körperorientierung des Anwenders zwischen einer aufrechteren Stellung und einer weiter zurückgelehnten Stellung des Körpers des Anwenders entspricht.

8. Gerät nach einem der vorhergehenden Ansprüche, des Weiteren umfassend einen Ausgabewandler (110, 210, 1430), wobei der durch die Steuerung (1250) gesteuerte Betrieb Ändern einer Verstärkung eines Schalls umfasst, der durch den Ausgabewandler (110, 210, 1430) erzeugt wird.

9. Gerät nach Anspruch 7 und 8, wobei der Betrieb Reduzieren der Verstärkung umfasst, wenn die Orientierungsdaten die Änderung der räumlichen Orientierung angeben, die der weiter zurückgelehnten Stellung des Körpers des Anwenders entspricht.

10. Gerät nach einem der vorhergehenden Ansprüche, wobei der Betrieb ein erster Betrieb ist, wobei die Steuerung (1250) ausgestaltet ist, um einen zweiten Betrieb des Hörgeräts basierend auf den Geh-Daten zu steuern.

11. Gerät nach einem der vorhergehenden Ansprüche, wobei der Trägheitssensor (1300) einen Beschleunigungsmesser (1301) umfasst.

12. Verfahren zum Betreiben eines Hörgeräts, umfassend ein Gehäuse (211, 212), das ausgestaltet ist, an einer Trageposition an einem Ohr eines Anwenders getragen zu werden, wobei das Verfahren umfasst:
- Bereitstellen von Orientierungsdaten, die eine räumliche Orientierung des Gehäuses (211, 212) und/oder die Trageposition angeben,
- Bereitstellen von Geh-Daten, die eine Geh-Aktivität des Anwenders angeben;
- Bereitstellen von Kalibrierungsdaten, die eine Abweichung der räumlichen Orientierung des Gehäuses (211, 212) von einer Referenzorientierung in Abhängigkeit von den Geh-Daten angeben, wobei die Orientierungsdaten basierend auf den Kalibrierungsdaten bereitgestellt werden; und
- Steuern eines Betriebs des Hörgeräts basierend auf den Orientierungsdaten,
wobei der Betrieb Steuern einer Eigenschaft eines Strahlformers umfasst, der in das Hörgerät eingeschlossen ist, wobei das Hörgerät des Weiteren ein Hinter-dem-Ohr-Teil (201) umfasst, das ausgestaltet ist, um an einer Trageposition hinter einem Ohr eines Anwenders getragen zu werden, wobei das Hinter-dem-Ohr-Teil das Gehäuse umfasst.

## Revendications

1. Dispositif auditif comprenant un boîtier (211, 212) configuré pour être porté dans une position de port à une oreille d'un utilisateur, le dispositif auditif comprenant :
- un capteur inertiel (1300) inclus dans le boîtier (211, 212) ;
- un évaluateur d'orientation (1230) couplé de manière communicative au capteur inertiel (1301), l'évaluateur d'orientation (1230) étant configuré pour fournir des données d'orientation indicatives d'une orientation spatiale du boîtier (211, 212) et/ou de la position de port ;
- un évaluateur de marche (1210) configuré pour fournir des données de marche indiquant une activité de marche de l'utilisateur ;
- un dispositif d'étalonnage d'orientation (1220) configuré pour fournir des données d'étalonnage indiquant un écart de l'orientation spatiale du boîtier (211, 212) par rapport à une orientation de référence, le dispositif d'étalonnage d'orientation (1220) étant configuré pour fournir les données d'étalonnage en fonction des données de marche, l'évaluateur d'orientation (1230) étant configuré pour fournir les données d'orientation sur la base des données d'étalonnage ; et
- un dispositif de commande (1250) configuré pour commander un fonctionnement du dispositif auditif sur la base des données d'orientation,
- un dispositif de formation de faisceau, l'opération consistant à commander une propriété du dispositif de formation de faisceau ; et
- une partie derrière l'oreille (201) configurée pour être portée à une position de port derrière l'oreille d'un utilisateur, la partie derrière l'oreille comprenant ledit boîtier.

2. Dispositif selon la revendication 1, le dispositif d'étalonnage d'orientation (1220) étant configuré pour ne pas prendre en compte ledit écart par rapport à l'orientation de référence dans lesdites données d'étalonnage lorsque les données de marche sont inférieures à un seuil de ladite activité de marche.

3. Dispositif selon la revendication 1 ou 2, le dispositif d'étalonnage d'orientation (1220) étant configuré pour inclure, dans lesdites données d'étalonnage, des données indicatives dudit écart par rapport à l'orientation de référence pendant un temps prédéterminé.

4. Dispositif selon la revendication 3, le dispositif d'étalonnage d'orientation (1220) étant configuré pour inclure dans lesdites données d'étalonnage, des données indicatives d'une valeur moyenne dudit écart moyen sur ledit temps prédéterminé.

5. Dispositif selon l'une quelconque des revendications précédentes, le dispositif d'étalonnage d'orientation (1220) étant configuré pour estimer ladite orientation de référence dans une direction fixe par rapport à une force gravitationnelle agissant sur le dispositif auditif.

6. Dispositif selon la revendication 5, le dispositif de commande (1250) étant configuré pour commander ladite opération lorsque les données d'orientation sont indicatives d'un changement de l'orientation spatiale du boîtier et/ou de la position de port.

7. Dispositif selon la revendication 6, ledit changement de l'orientation spatiale étant sélectionné de telle sorte qu'il correspond à un changement d'une orientation du corps de l'utilisateur entre une pose plus verticale et une pose plus inclinée du corps de l'utilisateur.

8. Dispositif selon l'une quelconque des revendications précédentes, comprenant en outre un transducteur de sortie (110, 210, 1430), l'opération commandée par le dispositif de commande (1250) comprenant la modification d'une amplification d'un son produit par le transducteur de sortie (110, 210, 1430).

9. Dispositif selon les revendications 7 et 8, l'opération comprenant la réduction de ladite amplification lorsque les données d'orientation sont indicatives de ladite modification de l'orientation spatiale correspondant à ladite pose plus inclinée du corps de l'utilisateur.

10. Dispositif selon l'une quelconque des revendications précédentes, l'opération étant une première opération, le dispositif de commande (1250) étant configuré pour commander une deuxième opération du dispositif auditif sur la base des données de marche.

11. Dispositif selon l'une quelconque des revendications précédentes, le capteur inertiel (1300) comprenant un accéléromètre (1301).

12. Procédé de fonctionnement d'un dispositif auditif comprenant un boîtier (211, 212) configuré pour être porté à une position de port au niveau d'une oreille d'un utilisateur, le procédé comprenant :
- la fourniture de données d'orientation indiquant une orientation spatiale du boîtier (211, 212) et/ou de la position de port,
- la fourniture de données de marche indiquant une activité de marche de l'utilisateur ;
- la fourniture, en fonction des données de marche, de données d'étalonnage indiquant un écart de l'orientation spatiale du boîtier (211, 212) par rapport à une orientation de référence, les données d'orientation étant fournies sur la base des données d'étalonnage ; et
- la commande d'un fonctionnement du dispositif auditif sur la base des données d'orientation, le fonctionnement comprenant la commande d'une propriété d'un dispositif de formation de faisceau inclus dans le dispositif auditif, le dispositif auditif comprenant en outre une partie derrière l'oreille (201) configurée pour être portée à une position de port derrière une oreille d'un utilisateur, la partie derrière l'oreille comprenant ledit boîtier.
